Europäisches Patentamt

European Patent Office

Office européen des brevets

(19)

(11) Numéro de publication: **0 225 833**
**A2**

(12)
# DEMANDE DE BREVET EUROPEEN

(21) Numéro de dépôt: 86402702.4

(22) Date de dépôt: 05.12.86

(51) Int. Cl.⁴: **A 61 K 35/78**
**A 61 K 37/22**

(30) Priorité: 06.12.85 FR 8518087

(43) Date de publication de la demande:
16.06.87 Bulletin 87/25

(84) Etats contractants désignés:
AT BE CH DE ES GB GR IT LI LU NL SE

(71) Demandeur: **Desjonqueres, Stéphane**
**7 rue du Chant des Oiseaux**
**F-78360 Montesson (Les Yvelines) (FR)**

(72) Inventeur: **Desjonqueres, Stéphane**
**7 rue du Chant des Oiseaux**
**F-78360 Montesson (Les Yvelines) (FR)**

(74) Mandataire: **Herrburger, Pierre**
**Cabinet Pierre Herrburger 115, boulevard Haussmann**
**F-75008 Paris (FR)**

(54) **Composition destinée à être utilisée en médecine vétérinaire, en particulier pour le traitement des affections de l'appareil locomoteur ou pour la préparation à l'effort et la récupération par exemple chez le cheval de sport ou le chien de travail, notamment de chasse.**

(57) a) Composition destinée à être utilisée en médecine vétérinaire, en particulier pour le traitement des affections de l'appaeil locomoteur ou pour la préparation à l'effort et la récupération par exemple chez le cheval de sport ou le chien de travail, notamment de chasse :

b) caractérisée en ce qu'elle contient au moins une huile naturelle d'origine végétale hyperoxygénée par saturation en oxygène et exposition intensive et contrôlée aux ultraviolets de façon à présenter un taux de péroxydation compris entre 30 et 300 exprimé en milli-équivalents et une teneur en glycérides oxydés comprise entre 5 et 40, la définition de cette huile en spectrophotométrie UV étant de 1 à 6 pour le facteur E 270 et de 8 à 60 pour le facteur E 232.

EP 0 225 833 A2

## Description

"Composition destinée à être utilisée en médecine vétérinaire, en particulier pour le traitement des affections de l'appareil locomoteur ou pour la préparation à l'effort et la récupération par exemple chez le cheval de sport ou le chien de travail, notamment de chasse "

La présente invention se rapporte à une composition destinée à être utilisée en médecine vétérinaire, en particulier pour le traitement des affections de l'appareil locomoteur traumatiques ou non, ainsi que pour la préparation à l'effort et la récupération, par exemple chez le cheval de sport ou le chien de travail, notamment de chasse.

La traumatologie et la rhumatologie sont des chapitres importants de la médecine vétérinaire notamment chez les chevaux, les chiens et les chats ; parmi les affections que les spécialistes sont le plus souvent amenés à traiter, on peut mentionner les affections musculaires, articulaires en tendineuses ou les manisfestations de fatigue chez les bêtes de sport.

Ces diverses affections sont le plus souvent traitées par des massages au moyen de produits relativement toxiques, par exemple à base de cortisone ou d'anti-inflamatoires non stéroïdiens du type phényl-butanol.

En plus de cette toxicité, il convient de mentionner que dans le traitement des tendinites et des traumatismes, du cheval en particulier, les éleveurs et propriétaires sont soucieux d'éviter toutes médications pouvant entraîner un verdict de dopage ; les cortisones font partie de ces médications.

La présente invention a pour but de remédier aux inconvénients susmentionnés en proposant une composition destinée à la médecine vétérinaire basée sur des propriétés surprenantes que certains corps gras hyperoxygénés ont révélé posséder.

La possibilité d'utiliser des péroxydes de corps gras pour le traitement de certaines affections en médecine humaine a été mentionnée pour la première fois dans le brevet français n° 2 330 M ; la poursuite de ces recherches a permis de mettre en évidence des qualités pharmacologiques des huiles hyperoxygénées, pouvant déboucher sur un certain nombre d'applications. Le brevet français n° 2 461 744 décrit, par exemple, un procédé de péroxydation de matières grasses pouvant aboutir à la préparation de compositions utilisables dans le domaine médical.

Conformément à l'invention, on s'est aperçu, de manière surprenante, que certains corps gras péroxydés appartenant à la famille susmentionnée, possédaient des qualités propres leur permettant de se montrer nettement supérieurs aux médicaments analogues utilisés jusqu'à présent dans certaines indications de la médecine vétérinaire.

A cet effet, l'invention concerne une composition renfermant de tels corps gras.

Cette composition est caractérisée en ce qu'elle contient au moins une huile naturelle d'origine végétale hyperoxygénée par saturation en oxygène et exposition intensive et contrôlée aux ultraviolets fe façon à présenter un taux de péroxydation compris entre 30 et 300 exprimé en milli-équivalents et une teneur en glycérides oxydés comprise entre 5 et 40, la définition de cette huile en spectrophotométrie UV étant de 1 à 6 pour le facteur E 270 et de 8 à 60 pour le facteur E 232. (NF T 60 223).

Les qualités particulières de cette composition qui renferme uniquement des produits naturels, sont basées sur une triple activité anti-inflammatoire, antalgique et cicatrisante.

L'activité anti-inflammatoire a été mise en évidence à partir d'un protocole expérimental traditionnel d'irritation locale de l'oreille du rat avec l'huile de croton ; au cours de ces expériences, l'activité de la composition objet de l'invention a été comparée avec celle de l'indométacine qui est l'anti-inflammatoire utilisé classiquement ; cette étude réalisée chez le rat a permis de conclure que l'administration topique de la composition objet de l'invention était capable de limiter de façon nette la phlogose induite localement par un agent irritant.

L'activité antalgique de cette composition a pu être démontrée par massage de témoins avec des huiles hyperoxygénées conformes à l'invention dans le cadre d'études cliniques en double aveugle, où le produit de comparaison était un anesthésiant de contact clairement défini par la pharmacopée française.

Des études cliniques réalisées en double aveugle contre placebo et d'autres travaux réalisés en ouvert, ont permis de mettre en lumière une activité cicatrisante indéniable, commune à toutes les huiles d'origine végétale hyperoxygénées répondant à la définition ci-dessus.

Selon une autre caractéristique de l'invention, la ou les huile(s) hyperoxygénée(s) est(sont) choisie(s) dans le groupe formé par l'huile d'arachide, l'huile d'amande douce et l'huile de carthame.

Il a été trouvé que ces huiles présentent à un degré élevé, la triple activité anti-inflammatoire, antalgique et cicatrisante susmentionnée, et, en même temps, se distinguent par une excellente tolérance et une absence totale de toxicité.

En effet, tous les travaux effectués dans ce sens (détermination de l'indice d'irritation primaire cutanée chez le lapin, détermination de l'indice d'irritation occulaire, étude de la toxicité transmuqueuse chez le lapin, recherche d'éventuels pouvoirs allergènes...) ont amené sans exception à conclure par un constat d'absence totale d'irritation cutanée ou de toute autre tolérance allergique ou non, qu'il s'agisse de la peau ou des muqueuses.

Parmi les huiles hyperoxygénées mentionnées ci-dessus, on a trouvé que l'huile d'arachide hyperoxygénée possédait des propriétés antalgiques et anti-inflammatoires en faisant un produit particulièrement actif dans les traitements suivants : rééducation post-chirurgicale, traitement des traumas de tissus mous, parésie et paralysie du train arrière, prévention des boiteries de fatigue chez les chiens de travail, en particulier de chasse, affections musculaires, articulaires ou tendineuses, notamment chez le cheval de sport...

Cette activité remarquable est due à la présence dans l'huile d'arachide, d'acide arachidonique, acide gras dont la structure est semblable aux médiateurs lipidiques de l'inflammation ; il est possible que cet acide agisse par compétition au niveau des sites récepteurs de ces médiateurs, diminuant ainsi l'inflammation locale.

Des travaux cliniques vétérinaires de la pathologie ostéo-articulaire et tendineuse ont démontré que l'action anti-inflammatoire et antalgique des huiles susmentionnées pouvait prendre une place nouvelle dans la panoplie des médicaments vétérinaires de premier choix.

Bien entendu, la composition objet de l'invention peut n'être constituée que par une ou plusieurs huiles naturelles d'origine végétale hyperoxygénées répondant à la définition mentionnée ci-dessus : cependant, et selon une autre caractéristique de l'invention, cette composition se présente également sous la forme de gel ; une composition ayant donné entière satisfaction contenait 93 % d'huile d'arachide hyperoxygénée et 7 % d'essence de romarin.

Il est essentiel de mentionner que les compositions objet de l'invention ne sont pas limitées à la présence des huiles susmentionnées, et que toutes les huiles végétales péroxydées répondant à la définition ci-dessus peuvent par faitement être utilisées dans le cadre des compositions conformes à l'invention.

L'activité des compositions conformes à l'invention sera mise en lumière grâce aux exemples ci-dessous :

Exemple 1 :

Cet exemple est un compte-rendu d'un essai ayant pour but de tester l'efficacité de la composition conforme à l'invention, sous forme d'huile et de pommade, en traumatologie, dans la préparation à l'effort et dans la récupération du cheval de sport.

Cet essai a été effectué dans le cadre d'un centre d'équitation sportive. 16 chevaux ont été traités par la composition conforme à l'invention.

La répartition des animaux selon leur activité sportive était la suivante :
- Concours de saut d'obstacle ..... 5 chevaux,
- Concours complet d'équitation ... 7 chevaux,
- Course d'obstacle ............... 2 chevaux,
- Jeunes chevaux ................. 2 chevaux.

Les 16 chevaux traités souffraient de diverses manifestations algiques et/ou inflammatoires de l'appareil locomoteur se répartissant comme suit :
- Affections musculaires .......... 4 cas
- Affections articulaires ......... 4 cas
- Affections tendineuses .......... 5 cas
- Affections diverses ............. 3 cas.

4 chevaux ont été traités avec la composition sous forme de pommade, tandis que les 12 autres ont été traités avec la forme d'huile. L'application se faisait 2 fois par jour en massage long et doux.

La durée d'application s'est échelonnée de 72 heures à 10 jours dans un but thérapeutique et elle a été pratiquée juste avant ou juste après l'effort dans un but préparatoire ou récupératoire à l'effort.

L'emploi de cette composition a donné pleine satisfaction dans 13 des cas traités. 2 cas n'ont donné que des résultats moyennement satisfaisants et 1 seul n'a donné aucun résultat. L'activité d'installation est rapide et la durée d'action est intéressante.

Lors de l'application (par massage long et doux de la zone traitée de façon à favoriser la pénétration du produit qui est freinée par la présence des poils) aucune réaction d'intolérance n'a pu être notée et aucune réaction générale n'est apparue.

En conclusion, la composition conforme à l'invention, sous forme d'huile ou de pommade, utilisée dans le traitement d'affections traumatiques de l'appareil locomoteur ou dans la préparation ou la récupération musculaire de cheval de sport, a permis d'obtenir des résultats remarquables.

De très intéressants résultats ont été notés dans le traitement des réactions conséquentes à un traumatisme.

Dans le traitement des tendinites, l'application de la composition dès l'apparition de l'affection a atténué les réactions et la déformation du tendon.

L'application de la composition sous forme d'huile en massage sur les masses musculaires dorsales, lombaires et fessières améliore et facilite l'échauffement des animaux. Elle semble aider à la récupéraion musculaire après un effort.

Cet exemple a permis de démontrer que la composition conforme à l'invention, sous forme d'huile ou de gel, peut être considérée comme utile et intéressante en médecine vétérinaire équestre.

Exemple 2 :

cet exemple est un compte-rendu d'essai ayant pour but de tester la composition conforme à l'invention pour le traitement des affections de l'appareil locomoteur chez les chiens et les chats.

38 animaux ont été traités et suivis dans les cas suivants :
- Rééducation post-chirurgicale,
- Traitement des traumas des tissus mous,
- Parésie du train arrière,
- Affections chroniques,
- Prévention des boiteries de fatigue les chiens de travail en particulier, de chasse.

Dans le groupe de rééducation post-chirurgicale, les animaux traités par massage ont récupéré plus vite que ceux non traités présentant des lésions considérées comme identiques ; le traitement a permis de diminuer les phénomènes douloureux, de sauvegarder l'état musculaire et de rétablir une fonction rapidement.

Dans ce groupe, le traitement a permis d'obtenir :
- 75 % de très bons résultats,
- 17 % de bons résultats,
- 8 % de résultats moyens.

Dans le groupe de traumatismes des tissus mous, sans association avec des lésions osseuses, la composition conforme à l'invention a été appliquée par massage, ou à l'aide de compresses imbibées du produit et laissées en place plusieurs heures ; les applications ont été renouvelées deux fois par jour.

Ce traitement a permis d'obtenir 92 % de très bons et bons résultats, c'est-à-dire que les réactions locales et la douleur ont été très nettement diminuées par rapport à celles observées sur des animaux ayant subi des traumas considérés comme similaires.

Dans le groupe des parésies avec douleurs récidivantes, le traitement instauré dès le début de la crise a diminué celle-ci en durée et en intensité dans 66 % des cas environ ; ceci a été évalué sur de vieux animaux, sujets à des crises périodiques dont on connaît l'évolution sans traitement et avec des traitements divers.

Dans les affections chroniques de l'appareil locomoteur (douleurs à répétition. avec boiterie, arthrose. anciens opérés présentant des troubles fonctionnels de manière intermittente, lors de phénomènes inflammatoires...) l'application de la composition objet de l'invention par massage une ou deux fois par jour a diminué la durée de la crise et son intensité.

La comparaison des crises sur un même animal a permis de constater, quelques fois, des résultats spectaculaires, certains chiens semblant rechercher les massages. On a évalué les très bons résultats et bons résultats à 73 %.

La prévention des boiteries de fatigue chez les chiens de travail, en particulier de chasse, a été particulièrement efficace : des animaux n'ont pas ou presque pas accusé une journée de travail alors qu'ils le font d'une manière habituelle.

Là encore, l'amélioration s'est situé à plus de 60 %.

En conclusion, l'essai ci-dessus a permis d'obtenir 71 % de résultats très bons et bons, 16 % de résultats moyens et 13 % de résultats faibles ou nuls.

Dans l'immense majorité des cas, l'application de la composition n'a pas posé de problèmes aux maîtres, sauf sur quelques animaux particulièrement difficiles, n'acceptant ni massage ni pansement. Aucune réaction secondaire n'a été enregistrée.

## Revendications

1°) Composition destinée à être utilisée en médecine vétérinaire, en particulier pour le traitement des affections de l'appareil locomoteur ou pour la préparation à l'effort et la récupération par exemple chez le cheval de sport ou le chien de travail, notamment de chasse, caractérisée en ce qu'elle contient au moins une huile naturelle d'origine végétale hyperoxygénée par saturation en oxygène et exposition intensive et contrôlée aux ultraviolets de façon à présenter un taux de péroxydation compris entre 30 et 300 exprimé en milli-équivalents et une teneur en glycérides oxydés comprise entre 5 et 40, la définition de cette huile en spectrophotométrie UV étant de 1 à 6 pour le facteur E 270 et de 8 à 60 pour le facteur E 232.

2°) Composition selon la revendication 1, caractérisée en ce que la ou les huile(s) hyperoxygénée(s) est (sont) choisie(s) dans le groupe formé par l'huile d'arachide, l'huile d'amande douce et l'huile de carthame.

3°) Composition selon l'une quelconque des revendications 1 et 2, caractérisée en ce que l'huile hyperoxygénée est l'huile d'arachide.

4°) Composition selon l'une quelconque des revendications 1 à 3, caractérisée en ce qu'elle se présente sous la forme d'une pommade.

5°) Composition selon l'une quelconque des revendications 1 à 4, caractérisée en ce qu'elle comporte environ 93 % d'huile d'arachide hyperoxygénée et environ 7 % d'essence de romarin.